# EUROPEAN PATENT SPECIFICATION

(11) **EP 3 083 526 B1**
(45) Date of publication and mention of the grant of the patent: **07.08.2019**
(21) Application number: 14815726.6
(22) Date of filing: 18.12.2014
(51) Int. Cl.: C07B 59/00, C07B 63/00, A61K 51/04, A61K 51/08, A61K 51/12, B01D 15/20, B01D 15/22, B01D 15/32, B01D 15/42, C07K 1/13, C07K 1/14, C07K 14/00

(54) **PURIFICATION METHOD AND COMPOSITIONS**
REINIGUNGSVERFAHREN UND -ZUSAMMENSETZUNGEN
PROCÉDÉ ET COMPOSITIONS DE PURIFICATION

(30) Priority: 18.12.2013 GB 201322451
(43) Date of publication of application: 26.10.2016
(73) Proprietor: GE Healthcare Limited, Little Chalfont Buckinghamshire HP15 7AT (GB)
(72) Inventor: ENGELL, Torgrim, N-0401 Oslo (NO); MEIJER, Andreas, Richard, N-0401 Oslo (NO); KHAN, Imitiaz Ahmed, Amersham Buckinghamshire HP7 9LL (GB); NAIRNE, Robert James, Amersham Buckinghamshire HP7 9LL (GB); MCROBBIE, Graeme, Amersham Buckinghamshire HP7 9LL (GB)
(74) Representative: Bannan, Sally
(86) International application number: PCT/EP2014/078608
(87) International publication number: WO 2015/091881

(56) References cited:
- WO-A1-2013/174909
- US-A1- 2013 209 358

### SEQUENCE LISTING

<110> GE Healthcare Limited
<120> PURIFCATION METHOD AND COMPOSITIONS
<130> PZ1392 PCT
<140> PCT/EP2014/078608
   <141> 2014-12-18
<160> 5
<170> PatentIn version 3.5
<210> 1
   <211> 8
   <212> PRT
   <213> Homo sapiens
<400> 1
   Lys Cys Arg Gly Asp Cys Phe Cys
   1               5
<210> 2
   <211> 17
   <212> PRT
   <213> Homo sapiens
<220>
   <221> X
   <222> (2)..(2)
   <223> Asn or His or Tyr
<220>
   <221> X
   <222> (4)..(4)
   <223> Gly or Ser or Thr or Asn
<220>
   <221> X
   <222> (8)..(8)
   <223> Thr or Arg
<220>
   <221> X
   <222> (15)..(15)
   <223> Ala or Asp or Glu or Gly or Ser
<220>
   <221> X
   <222> (16)..(16)
   <223> Ser or Thr
<220>
   <221> X
   <222> (17)..(17)
   <223> Asp or Glu
<400> 2
   Cys Xaa Cys Xaa Gly Pro Pro Xaa Phe Glu Cys Trp Cys Tyr Xaa Xaa
   1               5                   10                  15
   Xaa
<210> 3
   <211> 19
   <212> PRT
   <213> Homo sapiens
<220>
   <221> X
   <222> (2)..(2)
   <223> Arg or Lys
<220>
   <221> X
   <222> (15)..(15)
   <223> Beta-Hydroxyaspartic acid
<400> 3
   Cys Xaa Gln Ser Cys Ser Phe Gly Pro Phe Thr Phe Val Cys Xaa Gly
   1               5                   10                  15
   Asn Thr Lys
<210> 4
   <211> 26
   <212> PRT
   <213> Homo sapiens
<400> 4
   Ala Gly Ser Cys Tyr Cys Ser Gly Pro Pro Arg Phe Glu Cys Trp Cys
   1               5                   10                  15
   Tyr Glu Thr Glu Gly Thr Gly Gly Gly Lys
               20                  25
<210> 5
   <211> 61
   <212> PRT
   <213> Homo sapiens
<400> 5
   Ala Glu Ala Lys Tyr Ala Lys Glu Met Arg Asn Ala Tyr Trp Glu Ile
   1               5                   10                  15
   Ala Leu Leu Pro Asn Leu Thr Asn Gln Gln Lys Arg Ala Phe Ile Arg
               20                  25                  30
   Lys Leu Tyr Asp Asp Pro Ser Gln Ser Ser Glu Leu Leu Ser Glu Ala
           35                  40                  45
   Lys Lys Leu Asn Asp Ser Gln Ala Pro Lys Val Asp Cys
       50                  55                  60

## Description

### Field of the Invention.

The present invention relates to the field of radiopharmaceuticals for *in vivo* imaging, in particular to a method of purifying a radiotracer which comprises ¹⁸F-labelled aminoxy-functionalised biological targeting moiety. The invention provides radioprotectant-containing radiopharmaceutical compositions of the tracers, as well as associated automated methods and cassettes.

### Background to the Invention.

WO 2004/080492 A1 discloses a method for radiofluorination of a biological targeting vector, comprising reaction of a compound of formula (I) with a compound of formula (II):

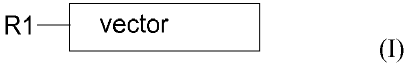

¹⁸F-(Linker)-R2 (II)

or,
a compound of formula (III) with a compound of formula (IV)

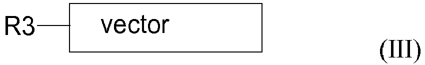

¹⁸F-(Linker)-R4 (IV)

wherein:
R1 is an aldehyde moiety, a ketone moiety, a protected aldehyde such as an acetal, a protected ketone, such as a ketal, or a functionality, such as diol or N-terminal serine residue, which can be rapidly and efficiently oxidised to an aldehyde or ketone using an oxidising agent;
R2 is a group selected from primary amine, secondary amine, hydroxylamine, hydrazine, hydrazide, aminoxy, phenylhydrazine, semicarbazide, and thiosemicarbazide;
R3 is a group selected from primary amine, secondary amine, hydroxylamine, hydrazine, hydrazide, aminoxy, phenylhydrazine, semicarbazide, or thiosemicarbazide;
R4 is an aldehyde moiety, a ketone moiety, a protected aldehyde such as an acetal, a protected ketone, such as a ketal, or a functionality, such as diol or N-terminal serine residue, which can be rapidly and efficiently oxidised to an aldehyde or ketone using an oxidising agent;
   to give a conjugate of formula (V) or (VI) respectively: 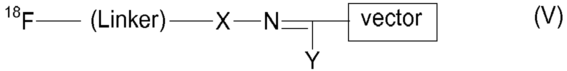 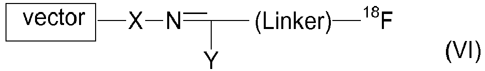
   wherein X is -CO-NH-, -NH-, -O-, -NHCONH-, or -NHCSNH-, and is
   preferably -CO-NH- , -NH- or -O- ; Y is a H, alkyl or aryl substituent; and
   the Linker group in the compounds of formulae (II), (IV), (V) and (VI) is selected from: 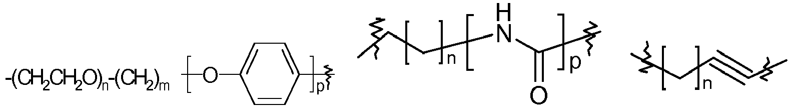 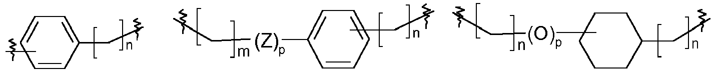 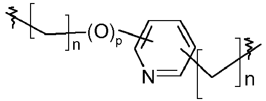 wherein:
   n is an integer of 0 to 20;
   m is an integer of 1 to 10;
   p is an integer of 0 or 1;
   Z is O or S.

Poethko et al [J.Nucl.Med., 45(5), 892-902 (2004)] disclose a method of radio labelling peptides with the radioisotope ¹⁸F, wherein an aminoxy-functionalised peptide is condensed with [¹⁸F]-fluorobenzaldehyde to give a labelled peptide having an oxime ether linkage as follows:

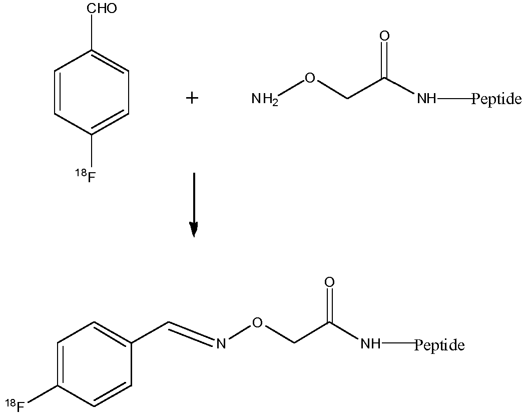

Schottelius et al [Bioconj.Chem., 19(6), 1256-1268 (2008)] further developed the method of Poethko *et al.* Schottelius *et al* use an aminoxy-functionalised peptide wherein the amine of the aminoxy group is protected with an *N-Boc* (Boc = *tert-*butyloxycarbonyl) protecting group. The desired aminoxy-functionalised peptide is generated *in situ* in the presence of [¹⁸F]-fluorobenzaldehyde *via* deprotection of the *N*-Boc group at acidic pH (pH = 2) at 75 °C. Schottelius *et al* used a 5-fold molar excess of the Boc-protected precursor, because the deprotection was not quantitative under the reaction conditions.

Mezo et al [J.Pept.Sci., 17, 39-46 (2010)] describe some of the problems associated with the above oxime ligation chemistry of Boc-protected aminoxy-functionalised peptides. Thus, it is known that the Boc-aminoxy reagent can acylate formed Boc-protected aminoxy-peptide, leading to undesirable by-products. It is also known that the reactivity of the free aminoxy group of the functionalised peptide is high towards carbonyl compounds. Consequently, unwanted condensation can occur with any adventitious aldehydes or ketones present either in the reaction mixture or in any subsequent purification steps. Such aldehydes or ketones could be traces of acetone present in the solvents used, or formaldehyde (e.g. from plasticizers). Mezo *et al* are interested in solving this problem for both the conjugation of anti-cancer drugs and of [¹⁸F]-fluorobenzaldehyde to peptides. Mezo *et al* solve the problem by carrying out the deprotection of the Boc-aminoxy peptide in the presence of a tenfold molar excess of free (aminoxy)acetic acid (Aoa) as a 'carbonyl capture agent'. The deprotected aminoxy-peptide and excess Aoa is then lyophilised and stored at 4 °C. Immediately prior to the oxime ligation reaction, the lyophilised mixture is reconstituted, and excess Aoa is separated by HPLC or Sep-Pak plus C18 cartridge. Mezo *et al* provide an example in which non-radioactive (i.e. ¹⁹F) 4-fluorobenzaldehyde is conjugated to an aminoxy-functionalised somatostatin peptide using this technique. Mezo *et al* do not provide any data on ¹⁸F-radiolabelling.

WO 2012/022676 discloses an imaging agent which comprises an ¹⁸F-radiolabelled 18 to 30-mer c-Met binding cyclic peptide of Formula I:

Z¹-[cMBP]-Z² (I)

where:
cMBP is of Formula II:

   -(A)ₓ-Q-(A')y- (II)

   where Q is the amino acid sequence (SEQ-1):

      -Cys^{a}-X¹-Cys^{c}-X²-Gly-Pro-Pro-X³-Phe-Glu-CyS^{d}-Trp-Cys^{b}-Tyr-X⁴-X⁵-X⁶-

      wherein X¹ is Asn, His or Tyr;
      X² is Gly, Ser, Thr or Asn;
      X³ is Thr or Arg;
      X⁴ is Ala, Asp, Glu, Gly or Ser;
      X⁵ is Ser or Thr;
      X⁶ is Asp or Glu;
      and Cys^{a-d} are each cysteine residues such that residues a and b as well as c and d are cyclised to form two separate disulfide bonds;
      A and A' are independently any amino acid other than Cys, with the proviso that at least one of A and A' is present and is Lys;
   x and y are independently integers of value 0 to 13, and are chosen such that [x + y] = 1 to 13;
Z¹ is attached to the N-terminus of cMBP, and is H or M^{IG};
Z² is attached to the C-terminus of cMBP and is OH, OB^{c}, or M^{IG},
   where B^{c} is a biocompatible cation;
   each M^{IG} is independently a metabolism inhibiting group which is a biocompatible group which inhibits or suppresses *in vivo* metabolism of the cMBP peptide;
wherein cMBP is labelled at the Lys residue of the A or A' groups with ¹⁸F.

WO 2012/022676 also discloses that the imaging agents may be used as pharmaceutical compositions, wherein said compositions preferably comprises one or more radioprotectants preferably chosen from: ethanol; ascorbic acid; *para-*aminobenzoic acid (i.e. 4-aminobenzoic acid or pABA); gentisic acid (i.e. 2,5-dihydroxybenzoic acid), and salts of such acids with a biocompatible cation.

WO 2012/072736 discloses the use of alternative protecting group chemistry for the aminoxy groups of functionalised biomolecules. The protected aminoxy group is of formula: 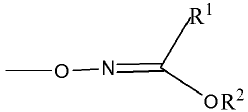 wherein:
R¹ and R² are independently chosen from C₁₋₃ alkyl, C₁₋₃ fluoroalkyl or C₄₋₆ aryl.

Lemaire et al [J.Lab.Comp.Radiopharm., 42, 63-75 (1999)] describe the solid phase extraction (SPE) purification of ¹⁸F-altanserin: 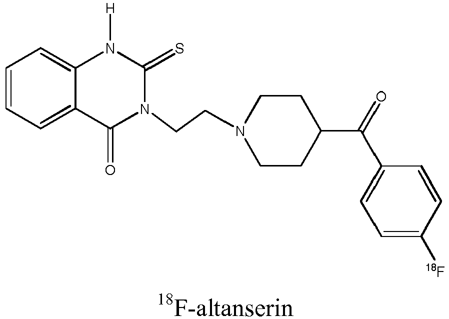 They noted that ¹⁸F-altanserin is very sensitive to radio lytic decomposition, and use a column conditioning solution of ethanol and saline with 0.1% ascorbic acid. The column washing was carried out with a saline/ascorbic acid mixture. The ¹⁸F-altanserin was eluted in neat ethanol, and subsequently diluted with the column conditioning solution.

US 2013/0209358 A1 discloses that ¹⁸F-fluciclatide can undergo radiolysis at high radioactive concentration:

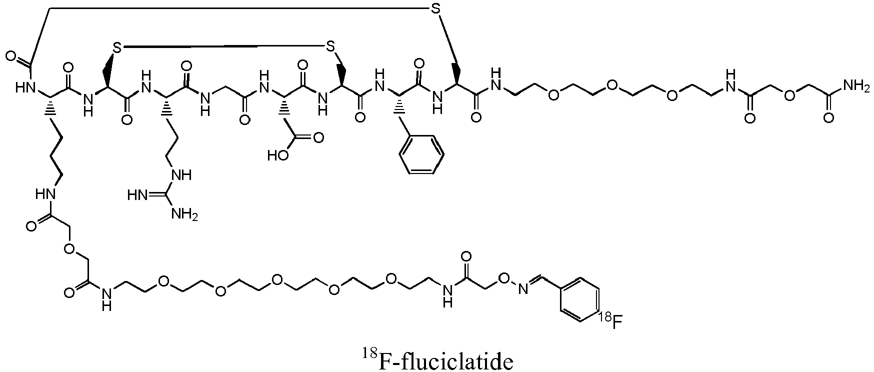

US 2013/0209358 A1 reports that ¹⁸F-fluciclatide is not stabilised by ethanol, and that ascorbic acid is not ideal for automated radiosyntheses, but that 4-aminobenzoic acid (or salt thereof) is effective. US 2013/0209358 A1 teaches that the ¹⁸F-fluciclatide is first prepared, and then the radioprotectant is added. US 2013/0209358 A1 also teaches radiopharmaceutical compositions comprising ¹⁸F-fluciclatide and the radioprotectant 4-aminobenzoic acid (pABA).

WO 2013/174909 A1 provides a method of purification of ¹⁸F-fluciclatide using SPE (solid phase extraction). WO 2013/174909 A1 teaches that, once eluted from the SPE column, the ¹⁸F-fluciclatide can be diluted with a biocompatible carrier, which can include 4-aminobenzoic acid.

There is therefore still a need for improved methods of preparing and purifying aminoxy-functionalised biological targeting moieties, to give high purity compositions suitable for radiopharmaceutical applications *in vivo.*

### The Present Invention.

The present inventors have identified and analysed the radiochemical impurities present in ¹⁸F-labelled aminoxy-functionalised biological targeting moieties, and how the levels of such impurities vary over time. That investigation led to the understanding that in-process radio lysis during attempted purification was the root cause of the RCP (radiochemical purity) problems.

This can be understood as follows. Thus, the radiotracers prepared according to the present invention, when purified and formulated for *in vivo* use, are present at a radioactive concentration (RAC) of approximately 500 to 700 MBq/mL (0.5 to 0.7 GBq/mL). The radiotracer may exhibit satisfactory stability, or minimal degradation under such RAC conditions. That is, however, obtained from a crude reaction mixture, where the RAC is in the range of ca. 10 to 15 GBq/mL. Furthermore, the present inventors have established that, during purification (i.e. at the end of the radiosynthesis), approximately 45 GBq of radioactivity is concentrated in a tight band on the chromatography column in a volume of less than 1 mL. That leads to an in-process RAC during purification of >45 GBq/mL. Since the purification can take up to 20 minutes, the risk of in-process radio lysis is high.

The present invention provides methods for stabilising the radiotracers whilst carrying out purification, and hence also provides improved radioactive yields and compositions. The present invention provides a method of purification of a radiotracer which comprises an ¹⁸F-aminoxy functionalised biological targeting moiety. It is in fact both a method of purification and a method of radiostabilisation (i.e. stabilising against radioactive degradation). Thus, by preventing in-process radiodegradation during purification, the present invention also improves the radiochemical purity, since impurities which might otherwise be generated are suppressed. The method also provides an improved radiochemical yield, since in-process losses during chromatography are minimised. Thus, compared to purification *via* HPLC without in-process radiostabilisation, the present invention provides an increase in yield of up to 65%.

### Detailed Description of the Invention.

In a first aspect, the present invention provides a method of purification of a radiotracer which comprises the following steps:
(a) provision of a radiotracer which comprises an ¹⁸F-labelled aminoxy-functionalised biological targeting moiety;
(b) adding a radioprotectant to said radiotracer to give a radiotracer solution which comprises said radiotracer in one or more aqueous water-miscible organic solvent(s) of 5 to 25% v/v organic solvent content;
(c) passing the radiotracer solution from step (b) through a reverse phase SPE cartridge, wherein the radiotracer is retained on said SPE cartridge;
(d) washing the SPE cartridge from step (c) one or more times with a wash solution which comprises an aqueous water-miscible organic solvent(s) solution of a radioprotectant of 15 to 25% v/v organic solvent content;
(e) washing the SPE cartridge from step (d) one or more times with water or aqueous buffer solution;
(f) eluting the washed SPE cartridge of step (d) or (e) with an elution solvent which comprises a radioprotectant in an aqueous ethanol solution having an ethanol content of 35 to 80 % v/v, wherein the eluent comprises purified radiotracer in said elution solvent;
wherein each radioprotectant independently comprises one or more of: ascorbic acid; *para*-aminobenzoic acid; and gentisic acid, and salts thereof with a biocompatible cation.

The term "radiotracer" has its' conventional meaning and refers to a radiopharmaceutical used to trace a physiological or biological process without affecting it. The term "radiopharmaceutical" has its' conventional meaning and refers to a radio labelled compound administered to the mammalian body *in vivo* for the purpose of imaging or therapy. During chromatographic purification, such as when loaded and hence concentrated into a small volume at the top of an SPE column, the radiotracer may transiently be exposed to very high radioactive concentration ("RAC"). Thus, radiotracers which otherwise appear radiostable, may exhibit instability with consequent loss of radiochemical purity ("RCP") and/or radiochemical yield during attempted purification.

The term "aminoxy-functionalised" means a biological targeting moiety functionalised with an aminoxy group. The term "aminoxy group" has its conventional meaning, and refers to a substituent of formula -O-NH₂, preferably -CH₂-O-NH₂.

By the term "biological targeting moiety" (BTM) is meant a compound which, after administration, is taken up selectively or localises at a particular site of the mammalian body *in vivo.* Such sites may for example be implicated in a particular disease state, or be indicative of how an organ or metabolic process is functioning.

The term "reverse phase" refers to "reverse phase chromatographic purification", which has its conventional meaning, and refers to chromatography where the stationary phase is lipophilic and the mobile phase is hydrophilic, typically involving aqueous media. The chromatographic technique of the present invention is solid phase extraction (SPE) using either an SPE column, sometimes called an 'SPE cartridge'. Such SPE columns have the advantage that they are single use, i.e. disposable, so there is no risk of cross-contamination with other radiotracers.

The term "aqueous water-miscible organic solvent(s) of 5 to 25% v/v organic solvent content" refers to an aqueous solution (e.g. water itself, saline, an aqueous buffer or mixtures thereof), mixed with at least one (i.e. possibly two or more different) water-miscible organic solvent(s). The 5 to 25% v/v organic solvent content can thus be from a single organic solvent, or two or more such solvents. Water-miscible organic solvents are known in the art, and include: acetonitrile, a C₁₋₄ alcohol, DMF, DMSO, dioxane, acetone, 2-methoxyethanol, THF and ethylene glycol.

By the term "radioprotectant" is meant a compound which inhibits degradation reactions, such as redox processes, by trapping highly-reactive free radicals, such as oxygen-containing free radicals arising from the radio lysis of water. The radioprotectants of the present invention are suitably chosen from: ascorbic acid, *para*-aminobenzoic acid (i.e. 4-aminobenzoic acid), gentisic acid (i.e. 2,5-dihydroxybenzoic acid) and salts thereof with a biocompatible cation. By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

The method of the present invention removes species which remain bound to the SPE column - e.g. very lipophilic species or any particulates. The method of the present invention also minimises or removes hydrophilic impurities which exhibit low affinity for the SPE column stationary phase - and are thus removed in the loading and washing steps. Such hydrophilic impurities include any salts or ionic species (such as fluoride ion); catalysts (such as aniline or Kryptofix); as well as the water-miscible organic solvent(s) from the radiotracer solution.

The radiotracer typically results from the conjugation of ¹⁸F-fluorobenzaldehyde (or similar) to an aminoxy-functionalised biological targeting moiety precursor, the conjugated fluorobenzaldehyde moiety confers additional lipophilicity to the conjugate. Hence, the radiotracer will tend to be retained on a reverse-phase SPE column, whereas the non-radioactive precursor itself (being more hydrophilic) will tend to be removed in the loading step (c) and washing steps (d) and (e) of the first aspect. Washing steps (d) and (e) are also important to remove the water-miscible organic solvent of step (b). In this way, the radiotracer solution is purified to remove unwanted non-radioactive impurities based on the biological targeting moiety which, if present, might compete with the radiotracer for biological sites of interest *in vivo.* Any more hydrophilic ¹⁸F-labelled, radioactive impurities will tend to be removed in a similar way. Thus, the initial level of Compound 2 in a preparation was 5 mg, which was reduced to ca. 200 µg (0.2 mg) in the purified radiotracer.

### Preferred features.

In the method of the first aspect, the water-miscible organic solvent of the radiotracer solution and/or the wash solution is preferably chosen from: acetonitrile, a C₂₋₄ alcohol, DMF, 2-methoxyethanol, THF and ethylene glycol. The water-miscible organic solvent is more preferably chosen from acetonitrile and ethanol.

Acetonitrile has the advantages that it is a good solvent for a wide range of radiotracers, is neither acidic nor basic, is relatively unreactive and thus compatible with a wide range of functional groups, and is highly miscible with water so that it is easily removed by the washing of the SPE column in method steps (d) and (e). Acetonitrile was found to be the most efficient solvent for removal of impurities in the radiotracer, including aniline. The acetonitrile content of the radiotracer solution and wash solution preferably does not exceed 25% v/v, since higher levels risk loss of radiotracer product by elution from the SPE column. The acetonitrile content of the wash solution is preferably 18-22 % v/v, more preferably 20-21% v/v.

The pH of the aqueous component of the radiotracer solution, wash solution, and elution solvent is preferably 7.5 to 8.5. That is preferably achieved using buffer solutions, more preferably phosphate buffer.

The radiotracer solution of step (b) preferably comprises ethanol, and more preferably comprises both acetonitrile and ethanol. Ethanol has potentially multiple roles, since it can function as: a water-miscible organic solvent (as defined above); a radioprotectant or radiostabiliser; a 'biocompatible carrier' (as defined below); and as an antimicrobial preservative (as defined below). The present inventors have found that the combination of the 'radioprotectant' (as defined above) and ethanol is most effective to stabilise the radiotracers of the invention against radiolysis. The ethanol content of the radiotracer solution is preferably 0.5 to 5% v/v ethanol.

The adding step (b) is preferably achieved by adding the wash solution as defined in step (d).

The radiotracer provided in step (a) and/or the radiotracer solution of step (b) are preferably cooled to a temperature range of 12 to 30 °C, more preferably 15 to 25 °C, most preferably 16 to 22 °C before use, in particular before loading onto the SPE column.

The wash solution of step (d) preferably comprises ethanol, and more preferably comprises both acetonitrile and ethanol. The ethanol content of the radiotracer solution is preferably 1.0 to 3%, more preferably 1.5-2.5 % and most preferably 2% v/v ethanol.

The reverse phase SPE cartridge of the first aspect preferably has a carbon load of 2.7 to 17%, and is more preferably a C8 or C18 SPE cartridge, most preferably a tC18 SPE cartridge.

The elution solvent of step (f) preferably comprises 35-70 %v/v aqueous ethanol, more preferably 40-60 %, most preferably 48-52% aqueous ethanol, and especially preferably 50% aqueous ethanol.

In the method of the first aspect, the radioprotectant used in the radiotracer solution, wash solution and elution solvent can be the same or different, or the same but used at different concentrations. Preferably, the radioprotectant used in the radiotracer solution, wash solution and elution solvent is the same. In that way, the presence of multiple different involatile components in the purified radiotracer is avoided, rendering it more suitable for *in vivo* applications. The radioprotectant of the first aspect preferably comprises 4-aminobenzoic acid, or a salt thereof with a biocompatible cation, more preferably sodium 4-aminobenzoate.

When the radioprotectant is sodium 4-aminobenzoate, a preferred concentration in the radiotracer solution is 5 mg/mL, and a preferred concentration in the wash solution and elution solvent is 2.5 mg/mL. An especially preferred radiotracer solution comprises 5 mg/mL sodium 4-aminobenzoate, 2% ethanol in a mixture of 79 g phosphate-buffered saline (pH 6 to 9, preferably 7 to 8.5) and 16.5 g acetonitrile. An especially preferred wash solution comprises 5 mg/mL sodium 4-aminobenzoate in phosphate-buffered saline (pH 6 to 9, preferably 7 to 8.5).

When the radioprotectant is sodium 4-aminobenzoate, the SPE cartridge is preferably flushed between purification steps with air instead of nitrogen. Thus, the present inventors have found that the radioprotectant 4-aminobenzoic acid functions more effectively in the presence of air, as opposed to the situation where oxygen is excluded.

The purified radiotracer of step (f) thus preferably contains 4-aminobenzoic acid, or a salt thereof with a biocompatible cation as radioprotectant, in 35-70% aqueous ethanol. As is described in the second and third aspects (below), for radiopharmaceutical applications that is preferably diluted with an aqueous biocompatible carrier to give a final ethanol content of 0.1 to 10% v/v.

The SPE cartridge of the first aspect is preferably conditioned prior to use by treating first with a water-miscible organic solvent, then with a conditioning solution. Said water-miscible organic solvent is preferably ethanol, and said conditioning solution is suitably an aqueous/water-miscible organic solvent mixture, preferably the wash solution of step (d).

The method of the first aspect is preferably carried out using an automated synthesizer apparatus as described in the third and fifth aspects (below).

In the method of the first aspect, the BTM preferably comprises a single amino acid, a 3-100 mer peptide, an enzyme substrate, an enzyme antagonist an enzyme agonist, an enzyme inhibitor or a receptor-binding compound. The BTM may be of synthetic or natural origin, but is preferably synthetic. The term "synthetic" has its conventional meaning, i.e. man-made as opposed to being isolated from natural sources e.g. from the mammalian body. Such compounds have the advantage that their manufacture and impurity profile can be fully controlled. Monoclonal antibodies and fragments thereof of natural origin are therefore outside the scope of the term 'synthetic' as used herein. The molecular weight of the BTM is preferably up to 30,000 Daltons. More preferably, the molecular weight is in the range 200 to 20,000 Daltons, most preferably 300 to 18,000 Daltons, with 400 to 16,000 Daltons being especially preferred. When the BTM is a non-peptide, the molecular weight of the BTM is preferably up to 3,000 Daltons, more preferably 200 to 2,500 Daltons, most preferably 300 to 2,000 Daltons, with 400 to 1,500 Daltons being especially preferred.

More preferably, BTM comprises either an Affibody™ or a single amino acid, a 3-100 mer peptide, an enzyme substrate, an enzyme antagonist an enzyme agonist, an enzyme inhibitor or a receptor-binding compound.

By the term "peptide" is meant a compound comprising two or more amino acids, as defined below, linked by a peptide bond (ie. an amide bond linking the amine of one amino acid to the carboxyl of another). The term "peptide mimetic" or "mimetic" refers to biologically active compounds that mimic the biological activity of a peptide or a protein but are no longer peptidic in chemical nature, that is, they no longer contain any peptide bonds (that is, amide bonds between amino acids). Here, the term peptide mimetic is used in a broader sense to include molecules that are no longer completely peptidic in nature, such as pseudo-peptides, semi-peptides and peptoids. The term "peptide analogue" refers to peptides comprising one or more amino acid analogues, as described below. See also Synthesis of Peptides and Peptidomimetics, M. Goodman et al, Houben-Weyl E22c, Thieme.

By the term "amino acid" is meant an L- or D-amino acid, amino acid analogue (eg. naphthylalanine) or amino acid mimetic which may be naturally occurring or of purely synthetic origin, and may be optically pure, i.e. a single enantiomer and hence chiral, or a mixture of enantiomers. Conventional 3-letter or single letter abbreviations for amino acids are used herein. Preferably the amino acids of the present invention are optically pure. By the term "amino acid mimetic" is meant synthetic analogues of naturally occurring amino acids which are isosteres, i.e. have been designed to mimic the steric and electronic structure of the natural compound. Such isosteres are well known to those skilled in the art and include but are not limited to depsipeptides, retro-inverso peptides, thioamides, cycloalkanes or 1,5-disubstituted tetrazoles [see M. Goodman, Biopolymers, 24, 137, (1985)]. Radiolabelled amino acids such as tyrosine, histidine or proline are known to be useful *in vivo* imaging agents.

Affibody™ molecules are based on the 58 amino acid residue domain derived from one of the IgG-binding domains of staphylococcal protein A. Affibodies may be used in monomer or dimer form, and have been reviewed by Nygren [FEBS J., 275, 2668-2676 (2008)] and Nilsson et al [Curr.Opin.Drug.Disc.Dev., 10, 167-175 (2007)]. The relatively small size of these Affibodies should allow better target tissue penetration and blood clearance compared to antibodies which are 10 to 20 times larger (∼150 kDa). Affibodies also have the advantage that they are stable under a range of pH conditions (pH 5.5 to 11). A preferred Affibody of the invention targets HER2. A preferred HER2 targeting Affibody comprises Affibody 1, as described below.

When the BTM is an enzyme substrate, enzyme antagonist, enzyme agonist, enzyme inhibitor or receptor-binding compound it is preferably a non-peptide, and more preferably is synthetic. By the term "non-peptide" is meant a compound which does not comprise any peptide bonds, ie. an amide bond between two amino acid residues. Suitable enzyme substrates, antagonists, agonists or inhibitors include glucose and glucose analogues; fatty acids, or elastase, Angiotensin II or metalloproteinase inhibitors. Suitable synthetic receptor-binding compounds include estradiol, estrogen, progestin, progesterone and other steroid hormones; ligands for the dopamine D-1 or D-2 receptor, or dopamine transporter such as tropanes; and ligands for the serotonin receptor.

The BTM is most preferably a 3-100 mer peptide or peptide analogue. When the BTM is a peptide, it is preferably a 4-30 mer peptide, and most preferably a 5 to 28-mer peptide.

When the BTM is an enzyme substrate, enzyme antagonist, enzyme agonist or enzyme inhibitor, preferred such biological targeting moieties of the present invention are synthetic, drug-like small molecules i.e. pharmaceutical molecules. Preferred dopamine transporter ligands such as tropanes; fatty acids; dopamine D-2 receptor ligands; benzamides; amphetamines; benzylguanidines, iomazenil, benzofuran (IBF) or hippuric acid.

When the BTM is a peptide, preferred such peptides include Peptide A, Peptide B, Peptide C and Peptide D as defined below, as well as:
- somatostatin, octreotide and analogues,
- peptides which bind to the ST receptor, where ST refers to the heat-stable toxin produced by *E.coli* and other micro-organisms;
- bombesin;
- vasoactive intestinal peptide;
- neurotensin;
- laminin fragments eg. YIGSR, PDSGR, IKVAV, LRE and KCQAGTFALRGDPQG,
- N-formyl chemotactic peptides for targeting sites of leucocyte accumulation,
- Platelet factor 4 (PF4) and fragments thereof,
- peptide fragments of α₂-antiplasmin, fibronectin or beta-casein, fibrinogen or thrombospondin. The amino acid sequences of α₂-antiplasmin, fibronectin, beta-casein, fibrinogen and thrombospondin can be found in the following references: α₂-antiplasmin precursor [M.Tone et al., J.Biochem, 102, 1033, (1987)]; beta-casein [L.Hansson et al, Gene, 139, 193, (1994)]; fibronectin [A.Gutman et al, FEBS Lett., 207, 145, (1996)]; thrombospondin-1 precursor [V.Dixit et al, Proc. Natl. Acad. Sci., USA, 83, 5449, (1986)]; R.F.Doolittle, Ann. Rev. Biochem., 53, 195, (1984);
- peptides which are substrates or inhibitors of angiotensin, such as:
   angiotensin II Asp-Arg-Val-Tyr-Ile-His-Pro-Phe (E. C. Jorgensen et al, J. Med. Chem., 1979, Vol 22, 9, 1038-1044)
   [Sar, Ile] Angiotensin II: Sar-Arg-Val-Tyr-Ile-His-Pro-Ile (R.K. Turker et al., Science, 1972, 177, 1203).
- Angiotensin I: Asp-Arg-Val-Tyr-Ile-His-Pro-Phe-His-Leu.

More preferred BTM peptides are chosen from Peptide A, Peptide B, Peptide C and Peptide D as defined below:
(i) Peptide A = an Arg-Gly-Asp peptide;
(ii) Peptide B = an Arg-Gly-Asp peptide which comprises the fragment 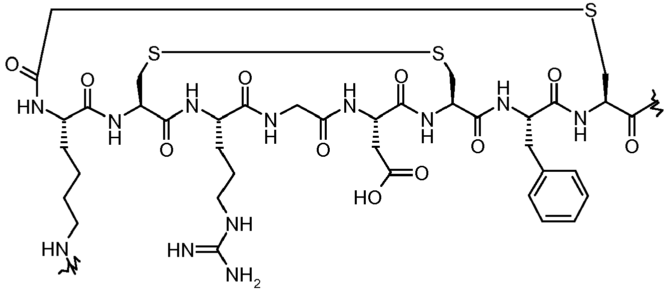
(iii) Peptide C = a c-Met binding cyclic peptide which comprises the amino acid sequence:
   -Cys^{a}-X¹-Cys^{c}-X²-Gly-Pro-Pro-X³-Phe-Glu-Cys^{d}-Trp-Cys^{b}-Tyr-X⁴-X⁵-X⁶-
   wherein X¹ is Asn, His or Tyr;
   X² is Gly, Ser, Thr or Asn;
   X³ is Thr or Arg;
   X⁴ is Ala, Asp, Glu, Gly or Ser;
   X⁵ is Ser or Thr;
   X⁶ is Asp or Glu;
   and Cys^{a-d} are each cysteine residues such that residues a and b as well as c and d are cyclised to form two separate disulfide bonds;

(i) Peptide D = a lantibiotic peptide of formula:
   wherein Xaa is Arg or Lys;
   Cys^{a}-Thr^{a}, Ser^{b}-Cys^{b} and Cys^{c}-Thr^{c} are covalently linked *via* thioether bonds;
   Ser^{d}-Lys^{d} are covalently linked *via* a lysinoalanine bond;
   HO-Asp is β-hydroxyaspartic acid.

By the term "lysinoalanine bond" is meant that the epsilon amine group of the Lys residue is linked as an amine bond to the Ser residue shown *via* dehydration of the hydroxyl functional group of the Ser giving a -(CH₂)-NH-(CH₂)₄- linkage joining the two *alpha-carbon* atoms of the amino acid residues.

Especially preferred BTM peptides are Peptide B, Peptide C and Peptide D.

A most preferred such Peptide B peptide is of formula (A): 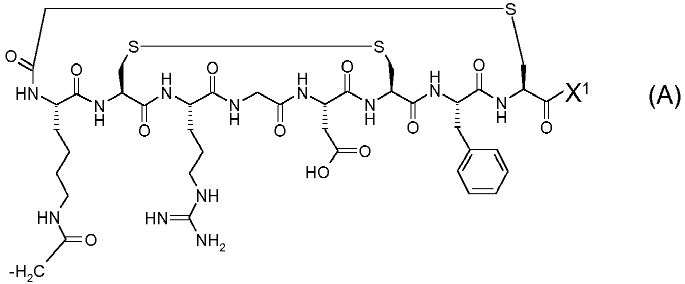 wherein X¹ is either -NH₂ or 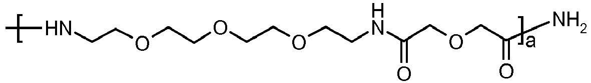 wherein a is an integer of from 1 to 10.

In Formula A, a is preferably 1.

A preferred c-Met binding cyclic peptide has the sequence:

When the BTM is a peptide, one or both termini of the peptide, preferably both, have conjugated thereto a metabolism inhibiting group (M^{IG}). Having both peptide termini protected in this way is important for *in vivo* imaging applications, since otherwise rapid metabolism would be expected with consequent loss of selective binding affinity for the BTM peptide. By the term "metabolism inhibiting group" (M^{IG}) is meant a biocompatible group which inhibits or suppresses enzyme, especially peptidase such as carboxypeptidase, metabolism of the BTM peptide at either the amino terminus or carboxy terminus. Such groups are particularly important for *in vivo* applications, and are well known to those skilled in the art and are suitably chosen from, for the peptide amine terminus:
N-acylated groups -NH(C=O)R^{G} where the acyl group -(C=O)R^{G} has R^{G} chosen from: C₁₋₆ alkyl, C₃₋₁₀ aryl groups or comprises a polyethyleneglycol (PEG) building block. Preferred such amino terminus M^{IG} groups are acetyl, benzyloxycarbonyl or trifluoroacetyl, most preferably acetyl.

Suitable metabolism inhibiting groups for the peptide carboxyl terminus include: carboxamide, tert-butyl ester, benzyl ester, cyclohexyl ester, amino alcohol or a polyethyleneglycol (PEG) building block. A suitable M^{IG} group for the carboxy terminal amino acid residue of the BTM peptide is where the terminal amine of the amino acid residue is *N*-alkylated with a C₁₋₄ alkyl group, preferably a methyl group. Preferred such M^{IG} groups are carboxamide or PEG, most preferred such groups are carboxamide.

Reverse phase SPE cartridges suitable for use in the present invention can be obtained from Waters Limited (730-740 Centennial Court, Centennial Park, Elstree, Hertfordshire, UK).

Aminoxy functionalised peptides can be prepared by the methods of Poethko et al [J.Nucl.Med., 45, 892-902 (2004)], Schirrmacher et al [Bioconj.Chem., 18, 2085-2089 (2007)], Indrevoll et al [Bioorg.Med.Chem.Lett, 16, 6190-6193 (2006)], Glaser et al [Bioconj. Chem., 19, 951-957 (2008)] or Dall'Angelo et al [Org.Biomol.Chem., 11, 4551-4558 (2013)]. The aminoxy group may optionally be conjugated in two steps. First, the *N*-protected aminoxy carboxylic acid or *N*-protected aminoxy activated ester is conjugated to the peptide (e.g. *via* conjugation to the amine group of a Lys residue, or *via* conventional solid phase synthesis). Second, the intermediate *N-*protected aminoxy functionalised peptide is deprotected to give the desired product [see Solbakken and Glaser papers cited above]. *N*-protected aminoxy carboxylic acids such as Boc-aminoxyacetic acid [Boc-NH-O-CH₂(C=O)OH] and Eei-N-O-CH₂(C=O)OH are commercially available, e.g. from Sigma-Aldrich, Novabiochem and IRIS.

The term "protected" refers to the use of a protecting group. By the term "protecting group" is meant a group which inhibits or suppresses undesirable chemical reactions, but which is designed to be sufficiently reactive that it may be cleaved from the functional group in question under mild enough conditions that do not modify the rest of the molecule. After deprotection the desired product is obtained. Amine protecting groups are well known to those skilled in the art and are suitably chosen from: Boc (where Boc is *tert*-butyloxycarbonyl); Eei (where Eei is ethoxyethylidene); Fmoc (where Fmoc is fluorenylmethoxycarbonyl); trifluoroacetyl; allyloxycarbonyl; Dde [i.e. 1-(4,4-dimethyl-2,6-dioxocyclohexylidene)ethyl] or Npys (i.e. 3-nitro-2-pyridine sulfenyl). The use of further protecting groups are described in Protective Groups in Organic Synthesis, 4th Edition, Theorodora W. Greene and Peter G. M. Wuts, [Wiley Blackwell, (2006)]. Preferred amine protecting groups are Boc and Eei, most preferably Eei.

In addition, the aminoxy-functionalised maleimide Mal-AO has been described by Padilla de Jesus et al [US 7,902,332 and Mol.Imaging Biol., 10, 177-181 (2008)]:

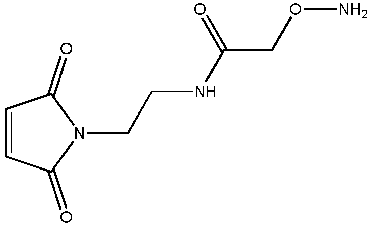

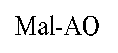

The bifunctional linker Mal-AO can be used to attach aminoxy functional groups to thiol-containing BTMs, by selective reaction of said thiol with the maleimide function of Mal-AO. Padilla de Jesus (cited above), apply this to the conjugation of Mal-AO to a HER2 selective Affibody.

In a second aspect, the present invention provides a method of preparation of a radiopharmaceutical composition, said composition comprising:
(i) the radiotracer as defined in the first aspect;
(ii) at least one radioprotectant as defined in the first aspect;
(iii) a biocompatible carrier which comprises aqueous ethanol having an ethanol content of 0.1 to 10 % v/v;
in a form suitable for mammalian administration;
where said method of preparation comprises:
- carrying out the radiotracer purification method of steps (a)-(f) as defined in the first aspect;
   (g) optionally diluting the purified [¹⁸F]-radiotracer from step (f) with a biocompatible carrier;
   (h) aseptic filtration of the optionally diluted solution from step (g) to give said [¹⁸F] radiopharmaceutical composition.

Preferred embodiments of the radiotracer and radioprotectant in the second aspect are as described in the first aspect (above).

The "biocompatible carrier" is a fluid, especially a liquid, in which the radioconjugate can be suspended or preferably dissolved, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is isotonic); an aqueous buffer solution comprising a biocompatible buffering agent (e.g. phosphate buffer); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols and the like). Preferably the biocompatible carrier is pyrogen-free water for injection, isotonic saline or phosphate buffer.

By the phrase "in a form suitable for mammalian administration" is meant a composition which is sterile, pyrogen-free, lacks compounds which produce toxic or adverse effects, and is formulated at a biocompatible pH (approximately pH 4.0 to 10.5). Such compositions lack particulates which could risk causing emboli *in vivo,* and are formulated so that precipitation does not occur on contact with biological fluids (e.g. blood). Such compositions also contain only biologically compatible excipients, and are preferably isotonic.

The radiotracer and biocompatible carrier are supplied in a suitable vial or vessel which comprises a sealed container which permits maintenance of sterile integrity and/or radioactive safety, plus optionally an inert headspace gas (e.g. nitrogen or argon), whilst permitting addition and withdrawal of solutions by syringe or cannula. A preferred such container is a septum-sealed vial, wherein the gas-tight closure is crimped on with an overseal (typically of aluminium). The closure is suitable for single or multiple puncturing with a hypodermic needle (e.g. a crimped-on septum seal closure) whilst maintaining sterile integrity. Such containers have the additional advantage that the closure can withstand vacuum if desired (eg. to change the headspace gas or degas solutions), and withstand pressure changes such as reductions in pressure without permitting ingress of external atmospheric gases, such as oxygen or water vapour.

Preferred multiple dose containers comprise a single bulk vial which contains multiple patient doses, whereby single patient doses can thus be withdrawn into clinical grade syringes at various time intervals during the viable lifetime of the preparation to suit the clinical situation. Pre-filled syringes are designed to contain a single human dose, or "unit dose" and are therefore preferably a disposable or other syringe suitable for clinical use.

The radiopharmaceutical composition may contain additional optional excipients such as: an antimicrobial preservative, pH-adjusting agent, filler, solubiliser or osmolality adjusting agent.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

By the term "solubiliser" is meant an additive present in the composition which increases the solubility of the agent of interest in the solvent. A preferred such solvent is aqueous media, and hence the solubiliser preferably improves solubility in water. Suitable such solubilisers include: C₁₋₄ alcohols; glycerine; polyethylene glycol (PEG); propylene glycol; polyoxyethylene sorbitan monooleate; sorbitan monooloeate; polysorbates; poly(oxyethylene)poly(oxypropylene)poly(oxyethylene) block copolymers (Pluronics™); cyclodextrins (e.g. alpha, beta or gamma cyclodextrin, hydroxypropyl-β-cyclodextrin or hydroxypropyl-γ-cyclodextrin) and lecithin.

By the term "antimicrobial preservative" is meant an agent which inhibits the growth of potentially harmful micro-organisms such as bacteria, yeasts or moulds. The antimicrobial preservative may also exhibit some bactericidal properties, depending on the dosage employed. The main role of the antimicrobial preservative(s) of the present invention is to inhibit the growth of any such micro-organism in the pharmaceutical composition. The antimicrobial preservative may, however, also optionally be used to inhibit the growth of potentially harmful micro-organisms in one or more components of kits used to prepare said composition prior to administration. Suitable antimicrobial preservative(s) include: the parabens, i.e. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the composition is within acceptable limits (approximately pH 4.0 to 10.5) for human or mammalian administration. Suitable such pH-adjusting agents include pharmaceutically acceptable buffers, such as tricine, phosphate or TRIS [i.e. *tris*(hydroxymethyl)aminomethane], and pharmaceutically acceptable bases such as sodium carbonate, sodium bicarbonate or mixtures thereof. When the composition is employed in kit form, the pH adjusting agent may optionally be provided in a separate vial or container, so that the user of the kit can adjust the pH as part of a multi-step procedure.

The method of the second aspect may be carried out in various ways:
1) aseptic manufacture techniques in which the steps are carried out in a clean room environment;
2) terminal sterilisation, in which steps (a)-(g) of the first aspect are carried out without using aseptic manufacture, and then sterilised as the last step [e.g. by gamma irradiation, autoclaving, dry heat or chemical treatment (e.g. with ethylene oxide)];
3) aseptic manufacture techniques in which the steps are carried out using an automated synthesizer apparatus.
Method (3) is preferred. Thus, in the method of the second aspect, at least one of steps (b)-(f) or (b)-(h) is preferably automated. More preferably, the automation is carried out using an automated synthesizer apparatus. Most preferably, the automated synthesizer apparatus comprises a single use cassette.

By the term "automated synthesizer" is meant an automated module based on the principle of unit operations as described by Satyamurthy et al [Clin.Positr.Imag., 2(5), 233-253 (1999)]. The term 'unit operations' means that complex processes are reduced to a series of simple operations or reactions, which can be applied to a range of materials. Such automated synthesizers are preferred for the method of the present invention especially when a radiopharmaceutical composition is desired. They are commercially available from a range of suppliers [Satyamurthy *et al,* above], including: GE Healthcare; CTI Inc; Ion Beam Applications S.A. (Chemin du Cyclotron 3, B-1348 Louvain-La-Neuve, Belgium); Raytest (Germany) and Bioscan (USA).

Commercial automated synthesizers also provide suitable containers for the liquid radioactive waste generated as a result of the radiopharmaceutical preparation. Automated synthesizers are not typically provided with radiation shielding, since they are designed to be employed in a suitably configured radioactive work cell. The radioactive work cell provides suitable radiation shielding to protect the operator from potential radiation dose, as well as ventilation to remove chemical and/or radioactive vapours. The automated synthesizer preferably comprises a cassette.

By the term "cassette" is meant a unit piece of apparatus designed such that the whole unit fits removably and interchangeably onto an automated synthesizer apparatus (as defined above), in such a way that mechanical movement of moving parts of the synthesizer controls the operation of the cassette from outside the cassette, i.e. externally. Suitable cassettes comprise a linear array of valves, each linked to a port where reagents or vials can be attached, by either needle puncture of an inverted septum-sealed vial, or by gas-tight, marrying joints. Each valve has a male-female joint which interfaces with a corresponding moving arm of the automated synthesizer. External rotation of the arm thus controls the opening or closing of the valve when the cassette is attached to the automated synthesizer. Additional moving parts of the automated synthesizer are designed to clip onto syringe plunger tips, and thus raise or depress syringe barrels.

The cassette is versatile, typically having several positions where reagents can be attached, and several suitable for attachment of syringe vials of reagents or chromatography cartridges (e.g. solid phase extraction or SPE). The cassette always comprises a reaction vessel. Such reaction vessels are preferably 1 to 10 cm³, most preferably 2 to 5 cm³ in volume and are configured such that 3 or more ports of the cassette are connected thereto, to permit transfer of reagents or solvents from various ports on the cassette. Preferably the cassette has 15 to 40 valves in a linear array, most preferably 20 to 30, with 25 being especially preferred. The valves of the cassette are preferably each identical, and most preferably are 3-way valves. The cassettes are designed to be suitable for radiopharmaceutical manufacture and are therefore manufactured from materials which are of pharmaceutical grade and ideally also are resistant to radio lysis.

Preferred automated synthesizers of the present invention comprise a disposable or single use cassette which comprises all the reagents, reaction vessels and apparatus necessary to carry out the preparation of a given batch of radio fluorinated radiopharmaceutical. The cassette means that the automated synthesizer has the flexibility to be capable of making a variety of different radiopharmaceuticals with minimal risk of cross-contamination, by simply changing the cassette. The cassette approach also has the advantages of: simplified set-up hence reduced risk of operator error; improved GMP (Good Manufacturing Practice) compliance; multi-tracer capability; rapid change between production runs; pre-run automated diagnostic checking of the cassette and reagents; automated barcode cross-check of chemical reagents vs the synthesis to be carried out; reagent traceability; single-use and hence no risk of cross-contamination, tamper and abuse resistance.

The method of the second aspect preferably further comprises:
(j) dispensing the [¹⁸F]-radiotracer radiopharmaceutical composition of step
(h) into one or more syringes.
The sequence step which follows step (h) is here deliberately chosen to be '(j)' - to avoid possible confusion with Roman numeral one.

In a third aspect, the present invention provides a single use cassette as defined in the second aspect for carrying out the automated method described therein, which cassette comprises:
(i) a vessel suitable for containing the [¹⁸F]-radiotracer solution to be purified as defined in the first aspect;
(ii) one or more reverse phase SPE cartridges as defined in the first aspect;
(iii) a supply of the wash solution as defined in the first aspect;
(iv) a supply of the elution solvent as defined in the first aspect wherein said elution solvent comprises 40-60 % v/v aqueous ethanol.

Preferred embodiments of the radiotracer, SPE cartridge, wash solution and elution solvent in the cassette are as described in the first aspect (above).

In a fourth aspect, the present invention provides the use of the cassette of the third aspect fitted onto an automated synthesizer apparatus to carry out the method of preparation of the first aspect, or the method of radiolabelling of the third aspect.

Preferred embodiments of the automated synthesizer in the fourth aspect are as described in the second aspect (above).

### Description of the Figures.

Figure 1 shows the radiation elution profile of Compound 3 through an SPE column, during the loading, washing and elution process using radioactivity detectors positioned throughout a FastLab cassette, including by side of the SPE column.
Figure 2 shows a FastLab cassette configuration for the automated radiosynthesis and automated purification of Compound 3.

The invention is illustrated by the non-limiting Examples detailed below. Example 1 provides the synthesis of a c-Met targeting peptide of the invention ("Peptide 1"). Example 2 provides the synthesis of an aminoxy-functionalised Peptide 1 ("Compound 1"), wherein the aminoxy functional group is protected with a protecting group (Eei), and subsequent deprotection to give Compound 2. Example 3 provides the radiosynthesis of [¹⁸F]-fluorobenzaldehyde. Example 4 is a comparative Example, which provides the radiosynthesis of the ¹⁸F-labelled conjugate Compound 3, without the methodology of the present invention. The RCP in this case is relatively low (79%) at the end of synthesis.

Example 5 provides an analysis of the identity and time-course of the radiochemical impurities in low RCP Compound 3 purified according to Example 4. This provides evidence that in-process radiolysis during attempted chromatographic purification was responsible for the low RCP. Example 5 provides information on the movement of radioactivity through the SPE column during SPE purification, demonstrating that the RAC is over 45 GBq/mL during the SPE process. This very high, but time-bound RAC, is also indicative of in-process radio lysis.

Example 7 provides an automated synthesis and purification of Compound 3, using an automated synthesizer and cassette. A significant increase in EOS yield was achieved by making up a MeCN/PBS purification solution containing 2.5 mg/Na-pABA, but the EOS RCP was still low, and vulnerable to high RAC (RCP= 89% when a RAC of 660 MBq/mL, RCP =85% when a RAC of 844 MBq/ml in a 25 mL formulation). A high RAC at EOS indicates yet higher RAC on the cartridge during the later stages of the purification process. The RCP was improved further by increasing the Na-pABA content of the radiotracer solution to 5 mg/mL to 89-91%. The addition of ethanol (2%) to the radiotracer solution and wash solution resulted in a further improvement in RCP to >92%. The process of Example 7 removes 85% of peptide-related impurities and essentially all of the aniline (20 µg remaining out of 100,000 µg aniline present in the crude product before purification). The addition of the pABA and ethanol did not adversely affect the performance of the SPE purification with respect to the removal of chemical impurities.

Example 8 provides the synthesis of a bifunctional aminoxy maleimide linker (Compound 4).

**Compounds of the Invention.**

| **Name** | **Structure** |
|---|---|
| Peptide 1 | Disulfide bridges at Cys4-16 and Cys6-14; |
| | |
| | or Ac-AGSCYCSGPPRFECWCYETEGTGGGK-NH₂ |
| Compound 1 | 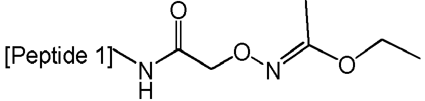 |
| Compound 2 | 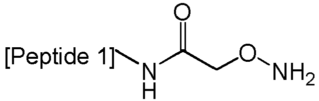 |
| Compound 3 | 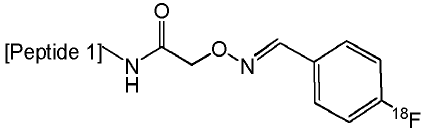 |
| Compound 4 | 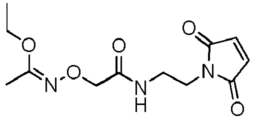 |
| Affibody 1 | |
| Precursor 1 | 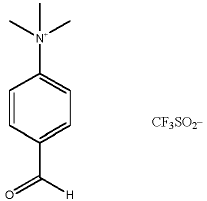 |

| | |
|---|---|
| where: Compounds 1, 2 and 3 are functionalised at the epsilon amine group of the carboxy terminal Lys of Peptide 1; Affibody 1 is selective for HER2. | |

### Abbreviations.

Conventional single letter or 3-letter amino acid abbreviations are used.
- Ac:: Acetyl
- Acm:: Acetamidomethyl
- ACN or MeCN:: Acetonitrile.
- AcOH:: Acetic acid.
- Boc:: *tert*-Butyloxycarbonyl.
- BTM:: biological targeting moiety.
- tBu:: *tertiary-*butyl
- DCM:: Dichloromethane
- DIPEA:: *N*,*N*-Diisopropylethyl amine
- DMF:: Dimethylformamide
- DMSO:: Dimethylsulfoxide
- Eei:: ethoxyethylidine;
- Eei-AOAc-OSu:: *N*-(1-Ethoxyethylidene)-2-aminoxyacetic acid *N-*hydroxysuccinimidyl ester;
- EOS:: end of synthesis;
- FBA:: 4-fluorobenzaldehyde;
- Fmoc:: 9-Fluorenylmethoxycarbonyl;
- HBTU:: *O*-Benzotriazol-1-yl-*N*,*N*,*N*',*N*'-tetramethyluronium hexafluorophosphate;
- HPLC:: High performance liquid chromatography;
- MW:: molecular weight;
- NHS:: *N*-hydroxy-succinimide;
- NMM:: *N-*Methylmorpholine;
- NMP:: 1-Methyl-2-pyrrolidinone;
- PBS:: phosphate-buffered saline;
- Pbf:: 2,2,4,6,7-Pentamethyldihydrobenzofuran-5-sulfonyl;
- RAC:: radioactive concentration.
- RCP:: radiochemical purity.
- RP-HPLC:: reverse-phase high performance liquid chromatography;
- tBu:: *tert-*butyl;
- TFA:: Trifluoroacetic acid;
- THF:: Tetrahydrofuran;
- TIS:: Triisopropylsilane;
- Trt:: Trityl.

### Example 1: Synthesis of Peptide 1.

### Step (a): synthesis of protected precursor linear peptide.

The precursor linear peptide has the structure: The peptidyl resin H-Ala-Gly-Ser(tBu)-Cys(Trt)-Tyr(tBu)-Cys(Acm)-Ser(tBu)-Gly-Pro-Pro-Arg(Pbf)-Phe-Glu(OtBu)-Cys(Acm)-Trp(Boc)-Cys(Trt)-Tyr(tBu)-Glu(OtBu)-Thr(ψ^{Me,Me}pro)-Glu(OtBu)-Gly-Thr(tBu)-Gly-Gly-Gly-Lys(Boc)-Polymer was assembled on an Applied Biosystems 433A peptide synthesizer using Fmoc chemistry starting with 0.1 mmol Rink Amide Novagel resin. An excess of 1 mmol pre-activated amino acids (using HBTU) was applied in the coupling steps. Glu-Thr pseudoproline (Novabiochem 05-20-1122) was incorporated in the sequence. The resin was transferred to a nitrogen bubbler apparatus and treated with a solution of acetic anhydride (1 mmol) and NMM (1 mmol) dissolved in DCM (5 mL) for 60 min. The anhydride solution was removed by filtration and the resin washed with DCM and dried under a stream of nitrogen.

The simultaneous removal of the side-chain protecting groups and cleavage of the peptide from the resin was carried out in TFA (10 mL) containing 2.5 % TIS, 2.5 % 4-thiocresol and 2.5 % water for 2 hours and 30 min. The resin was removed by filtration, TFA removed *in vacuo* and diethyl ether added to the residue. The formed precipitate was washed with diethyl ether and air-dried affording 264 mg of crude peptide.

Purification by preparative HPLC (gradient: 20-30 % B over 40 min where A = H₂O/0.1 % TFA and B = ACN/0.1 % TFA, flow rate: 10 mL/min, column: Phenomenex Luna 5µ C18 (2) 250 x 21.20 mm, detection: UV 214 nm, product retention time: 30 min) of the crude peptide afforded 100 mg of pure Peptide 1 linear precursor. The pure product was analysed by analytical HPLC (gradient: 10-40 % B over 10 min where A = H₂O/0.1 % TFA and B = ACN/0.1 % TFA, flow rate: 0.3 mL/min, column: Phenomenex Luna 3µ C18 (2) 50 x 2 mm, detection: UV 214 nm, product retention time: 6.54 min). Further product characterisation was carried out using electrospray mass spectrometry (MH₂²⁺ calculated: 1464.6, MH₂²⁺ found: 1465.1).

### Step (b): Formation of Monocyclic Cys4-16 disulfide bridge.

The linear precursor from step (a) (100 mg) was dissolved in 5 % DMSO/water (200 mL) and the solution adjusted to pH 6 using ammonia. The reaction mixture was stirred for 5 days. The solution was then adjusted to pH 2 using TFA and most of the solvent removed by evaporation *in vacuo.* The residue (40 mL) was injected in portions onto a preparative HPLC column for product purification.

Purification by preparative HPLC (gradient: 0 % B for 10 min, then 0-40 % B over 40 min where A = H₂O/0.1 % TFA and B = ACN/0.1 % TFA, flow rate: 10 mL/min, column: Phenomenex Luna 5µ C18 (2) 250 x 21.20 mm, detection: UV 214 nm, product retention time: 44 min) of the residue afforded 72 mg of pure Peptide 1 monocyclic precursor. The pure product (as a mixture of isomers P1 to P3) was analysed by analytical HPLC (gradient: 10-40 % B over 10 min where A = H₂O/0.1 % TFA and B = ACN/0.1 % TFA, flow rate: 0.3 mL/min, column: Phenomenex Luna 3µ C18 (2) 50 x 2 mm, detection: UV 214 nm, product retention time: 5.37 min (P1); 5.61 min (P2); 6.05 min (P3)). Further product characterisation was carried out using electrospray mass spectrometry (MH₂²⁺ calculated: 1463.6, MH₂²⁺ found: 1464.1 (P1); 1464.4 (P2); 1464.3 (P3)).

### Step (c): Formation of Second Cys6-14 disulfide bridge (Peptide 1).

The monocyclic precursor from step (b) (72 mg) was dissolved in 75 % AcOH/water (72 mL) under a blanket of nitrogen. 1 M HCl (7.2 mL) and 0.05 M I₂ in AcOH (4.8 mL) were added in that order and the mixture stirred for 45 min. 1 M ascorbic acid (1 mL) was added giving a colourless mixture. Most of the solvents were evaporated *in vacuo* and the residue (18 mL) diluted with water/0.1 % TFA (4 mL) and the product purified using preparative HPLC. Purification by preparative HPLC (gradient: 0 % B for 10 min, then 20-30 % B over 40 min where A = H₂O/0.1 % TFA and B = ACN/0.1 % TFA, flow rate: 10 mL/min, column: Phenomenex Luna 5µ C18 (2) 250 x 21.20 mm, detection: UV 214 nm, product retention time: 43-53 min) of the residue afforded 52 mg of pure Peptide 1. The pure product was analysed by analytical HPLC (gradient: 10-40 % B over 10 min where A = H₂O/0.1 % TFA and B = ACN/0.1 % TFA, flow rate: 0.3 mL/min, column: Phenomenex Luna 3µ C18 (2) 50 x 2 mm, detection: UV 214 nm, product retention time: 6.54 min). Further product characterisation was carried out using electrospray mass spectrometry (MH₂²⁺ calculated: 1391.5, MH₂²⁺ found: 1392.5).

### Example 2: Synthesis, Purification and Lyophilization of Compound 2.

Peptide 1 (0.797 g) and Eei-AOAc-OSu (IRIS Biotech; 127 mg) were dissolved in DMF (12 mL). DIPEA (100 µL) was added and the reaction mixture shaken for 26 min. A second aliquot of DIPEA (80 µL) was added and the reaction mixture shaken for 2 hr. The reaction mixture was then diluted with 10% ACN/water/0.1 % ammonium acetate (40 mL), and the product purified by preparative HPLC using A = 0.1% TFA/water and B = ACN with gradient elution of 20-40% B over 40 min. The fractions containing pure products (these are a mixture of Compound 1 and Compound 2) were pooled in a flask and the flask flushed with argon. The solution was stirred overnight to afford complete removal of Eei protecting groups. The deprotected product was lyophilised affording 550 mg (69% yield) of Compound 2.

The pure product was analysed by analytical LC-MS (gradient: 10-40 % B over 5 min where A = H₂O/0.1 % TFA and B = ACN TFA, flow rate: 0.6 mL/min, column: Phenomenex Luna 3µ C18 (2) 20 x 2 mm, detection: UV 214 nm, product retention time: 3.00 min), MH₂²⁺ calculated: 1428.1, MH₂²⁺ found: 1427.9).

### Example 3: Radiosynthesis of [¹⁸ F]-fluorobenzaldehyde (¹⁸F-FBA).

[¹⁸F]-fluoride was produced using a GEMS PETtrace cyclotron with a silver target *via* the [¹⁸O](p,n) [¹⁸F] nuclear reaction. Total target volumes of 3.2 - 4.8 mL were used. The radio fluoride was trapped on a Waters QMA cartridge (pre-conditioned with carbonate), and the fluoride is eluted with a solution of Kryptofix_{2.2.2.} (5.14 mg) and potassium bicarbonate (1.40 mg) in water (800 µL) and acetonitrile (200 µL). Nitrogen was used to drive the solution off the QMA cartridge to the reaction vessel. The [¹⁸F]-fluoride was dried for 9 minutes at 120 °C under a steady stream of nitrogen and vacuum. Trimethylammonium benzaldehyde triflate, [Precursor 1; Haka et al, J.Lab.Comp.Radiopharm., 27, 823-833 (1989)] (3.7 mg), in DMSO (2.0 mL) was added to the dried [¹⁸F]-fluoride, and the mixture heated to 80 °C for 2 minutes to produce 4-[¹⁸F]-fluorobenzaldehyde.

### Example 4: Radiosynthesis of Compound 3 (Comparative Example).

Compound 2 from Example 2 was radio labelled with ¹⁸F using ¹⁸F-FBA from Example 3, then purified using a MCX+ SPE column, without the in-process radiostabilisation of the prseent invention, giving Compound 3 with an RCP of 79%.

### Example 5: Radiochemical Impurities in Low RCP Compound 3.

The RCP of Compound 3 prepared as per Example 3 was studied as a function of time. The RCP did not drop further over time (up to 8 hours), showing that:
(i) Compound 3 is relatively radiostable at the RAC conditions existing at the end of the SPE process;
(ii) the RCP must already have been low at the end of the SPE process.

Analysis of Compound 3 from Example 4, i.e. without using the radiostabilisation methodology of the present invention and exhibiting low RCP (79%), found two radio lysis products to be the main contributors to the low RCP. These were identified by retention time on analytical HPLC, and comparison with the retention time of authentic samples of the non-radioactive analogues. These two radiolysis products are [¹⁸F]4-fluorobenzaldehyde (FBA) and [¹⁸F]4-fluorobenzonitrile (FPhCN), which together represented 12% of the radioactivity present in the low RCP Compound 3 preparation from Example 4.

These principal radiochemical impurities, which do not increase significantly with time, represent radiodegradation products of Compound 3, and in turn, in-process radiolysis.

### Example 6: SPE Elution Profile in the Purification of Compound 3.

Six radioactivity detectors were positioned along a FASTlab cassette, with Detector #6 positioned towards the bottom of the SPE column of a preparation according to Example 7. The movement of radioactivity during the loading, washing, and elution steps of the SPE purification process was followed in this way.

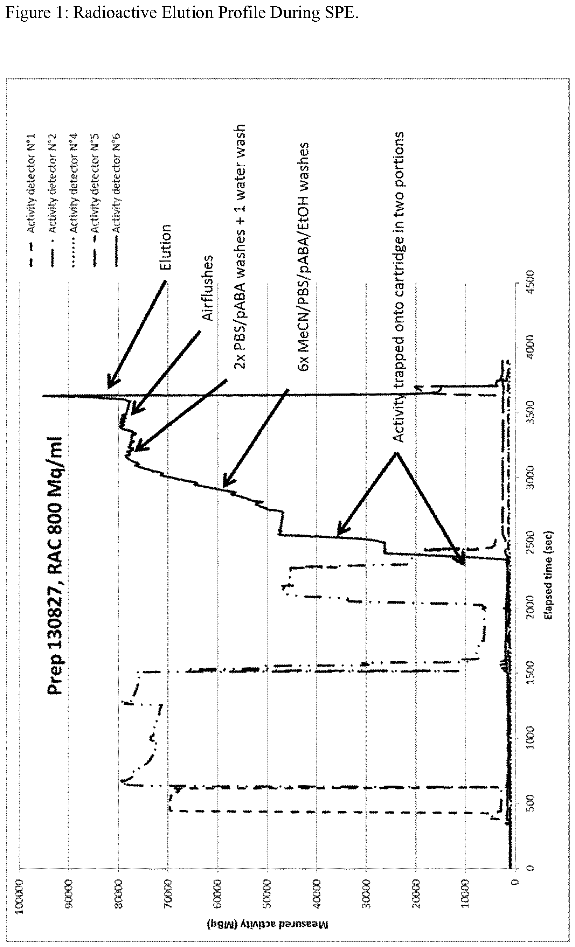

The results are shown in Figure 1. The crude product was shown to be trapped on the top of the SPE cartridge. As the purification proceeds, the radioactivity moved down the cartridge and towards detector 6, increasing the signal. This demonstrated that the radioactivity is not spread out over the entire cartridge, but concentrated in a tight band. During the purification, all the activity was concentrated into a volume of less than 1 mL, giving a RAC during purification (up to 20 min) of 45,000 MBq/mL, i.e. 45 GBq/mL.

### Example 7: Automated Synthesis and Purification of Compound 3.

A FASTlab automated synthesizer (GE Healthcare Ltd) with cassette was used. The tC18 cartridge was obtained from Waters Limited (address as above). Precursor 1 was reacted with [¹⁸F]-fluoride on the Fastlab according to Example 3 to give [¹⁸F]-FBA. The [¹⁸F]-FBA was reacted subsequently on the FastLab with Compound 2 (aminoxy derivative of Peptide 1), to give crude Compound 3.

### Purification.

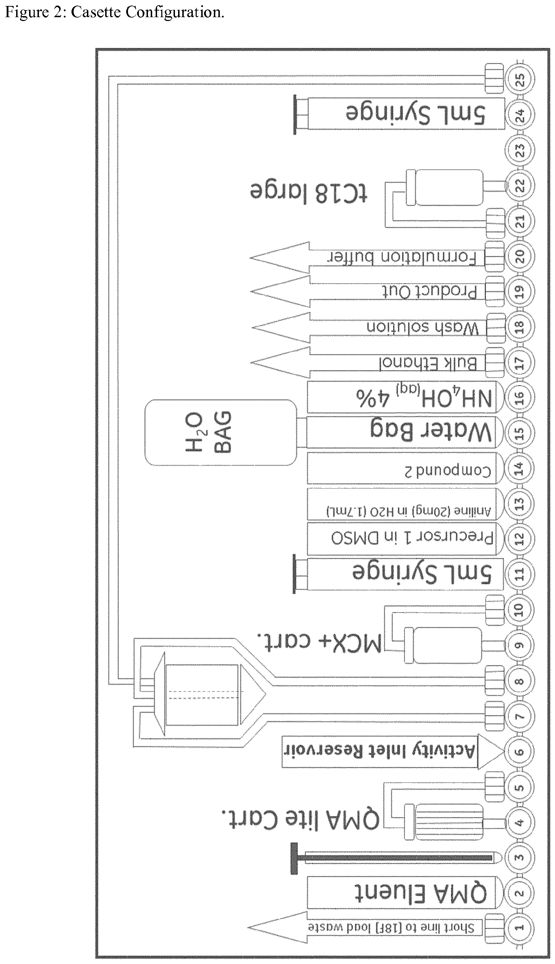

The cassette configuration is given in Figure 2. Three external solvent vials are used on the cassette for the SPE purification:
Position 17 = Anhydrous ethanol;
Position 18 = Wash Solution of 5 mg/mL Na-pABA 2% EtOH in 79 g PBS/16.5g MeCN;
Position 20 = Formulation Buffer of 34 mL PBS containing 80 mg Na-pABA.

Other cassette positions:
Position 21: Tubing to the tC18 cartridge in Position 22;
Position 22: tC18 cartridge;
Position 23: Sterile filter.

### FASTlab procedure.

In the following, P17 etc refers to Position 17 of the cassette. S2 and S3 refer to syringe 2 and syringe 3:
(i) the first part of the purification process was conditioning with full S2 fill with ethanol from P17, followed by a full S2 fill of MeCN/PBS solution from P18.
(ii) crude Compound 3 in the aqueous ethanol solution from the conjugation step was diluted 1:1 with the formulation buffer from P20. This was done in two portions: half of the content of the crude volume from the reaction vessel was transferred to S2, and thereafter mixed with the same volume of formulation buffer from P20. This mixture was then slowly trapped onto the tC18 cartridge. After the first trapping, the same procedure was repeated with the remaining half of the crude.
(iii) S2 was rinsed with water and thereafter a full fill of S2 with the MeCN wash solution from P18. The MeCN wash solution was slowly pushed through the tC18 cartridge and to waste. This was repeated 5 more times - six such washes in total (similar results were obtained when the procedure had just three washes in total).
(iv) the MeCN on the tC18 cartridge was removed by solvent exchange: first 2x full fill of S2 with the formulation buffer from P20 followed by one full fill of S2 with water from the water bag.
(v) the eluent was made by mixing of 3 mL ethanol from P17 and 3 mL of formulation buffer from P20 in S2. The first 1 mL of the eluent was passed through the tC18 and to waste, the following 4 mL eluent was passed through the tC18 and the purified Compound 3 product collected in S3. After elution the product was transferred out from the FASTlab and into the product vial through P19.

The radiochemical purity (RCP) in this case was 92%.

### Example 8: Synthesis of Bifunctional Linker (Compound 4).

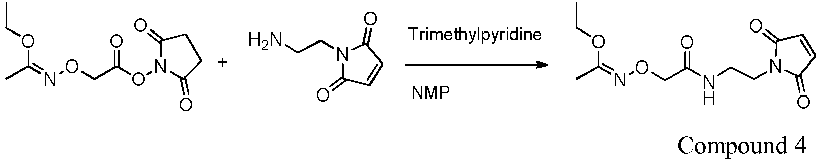

*N*-(2-aminoethyl)maleimide TFA-salt (Sigma-Aldrich; 151 mg.) and Eei-AOAc-OSu (IRIS Biotech;77 mg) were stirred in NMP (2 mL) at ambient temperature. Trimethylpyridine (80 µL) was added, and the reaction mixture stirred at ambient temperature for 70 min. The reaction was quenched by dilution with 0.1% acetic acid (7 mL). The product was purified by preparative HPLC as follows:

| | |
|---|---|
| Detection | UV at 214 nm and 254 nm |
| Column type and size | Luna C-18 (2), 5µm, 100Å, 20 x 250 mm from Phenomenex |
| Eluent A | 0.1% v/v acetic acid in water, 1 mL/L |
| Eluent B | Acetonitrile (Lichrosolv) |
| Gradient | 15-30% B during 40 min. |
| Flow rate | 10 ml/min during gradient elution |

The purified Compound 4 was freeze-dried. Yield 43 mg (75%), purity: > 97% by area.

The pure product was analysed by analytical LC-MS (gradient: 10-40 % B over 5 min where A = H₂O/0.1 % TFA and B = ACN/0.1 % TFA, flow rate: 0.6 mL/min, column: Phenomenex Luna 3µ C18 (2) 20 x 2 mm, detection: UV 214 nm, product retention time: 1.93 min), MH⁺ calculated: 284.1, MH⁺ found: 284.1).

## Claims

1. A method of purification of a radiotracer which comprises the following steps:
(a) provision of a radiotracer which comprises an ¹⁸F-labelled aminoxy-functionalised biological targeting moiety;
(b) adding a radioprotectant to said radiotracer to give a radiotracer solution which comprises said radiotracer in one or more aqueous water-miscible organic solvent(s) of 5 to 25% v/v organic solvent content;
(c) passing the radiotracer solution from step (b) through a reverse phase SPE cartridge, wherein the radiotracer is retained on said SPE cartridge;
(d) washing the SPE cartridge from step (c) one or more times with a wash solution which comprises an aqueous water-miscible organic solvent(s) solution of a radioprotectant of 15 to 25% v/v organic solvent content;
(e) washing the SPE cartridge from step (d) one or more times with water or aqueous buffer solution;
(f) eluting the washed SPE cartridge of step (d) or (e) with an elution solvent which comprises a radioprotectant in an aqueous ethanol solution having an ethanol content of 35 to 80 % v/v, wherein the eluent comprises purified radiotracer in said elution solvent;
wherein each radioprotectant independently comprises one or more of: ascorbic acid; *para*-aminobenzoic acid; and gentisic acid, and salts thereof with a biocompatible cation.

2. The method of claim 1, where the water-miscible organic solvent of the radiotracer solution comprises acetonitrile.

3. The method of claim 1 or claim 2, where the radiotracer solution of step (b) comprises 0.5 to 5% v/v ethanol.

4. The method of any one of claims 1 to 3, wherein the SPE cartridge is a C18 SPE cartridge.

5. The method of any one of claims 1 to 4 wherein the elution solvent of step (f) comprises 35 to 70 % v/v aqueous ethanol.

6. The method of any one of claims 1 to 5, where the radioprotectant comprises 4-aminobenzoic acid, or a salt thereof with a biocompatible cation.

7. The method of any one of claims 1 to 6, where the BTM comprises a single amino acid, a 3-100 mer peptide, an enzyme substrate, an enzyme antagonist an enzyme agonist, an enzyme inhibitor or a receptor-binding compound.

8. The method of claim 7, where the BTM comprises an Affibody™.

9. The method of claim 7, where the BTM comprises a 3-100 mer peptide which is chosen from Peptide A, Peptide B, Peptide C and Peptide D as defined below:
(i) Peptide A = an Arg-Gly-Asp peptide;
(ii) Peptide B = an Arg-Gly-Asp peptide which comprises the fragment 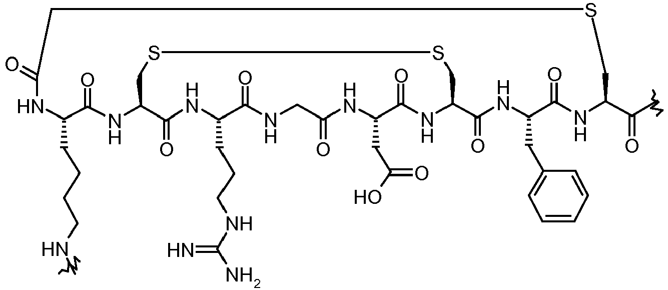
(iii) Peptide C = a c-Met binding cyclic peptide which comprises the amino acid sequence:
-Cys^{a}-X¹-Cys^{c}-X²-Gly-Pro-Pro-X³-Phe-Glu-Cys^{d}-Trp-Cys^{b}-Tyr-X⁴-X⁵-X⁶-
wherein X¹ is Asn, His or Tyr;
X² is Gly, Ser, Thr or Asn;
X³ is Thr or Arg;
X⁴ is Ala, Asp, Glu, Gly or Ser;
X⁵ is Ser or Thr;
X⁶ is Asp or Glu;
and Cys^{a-d} are each cysteine residues such that residues a and b as well as c and d are cyclised to form two separate disulfide bonds;
(iv) Peptide D = a lantibiotic peptide of formula:
wherein Xaa is Arg or Lys;
Cys^{a}-Thr^{a}, Ser^{b}-Cys^{b} and Cys^{c}-Thr^{c} are covalently linked *via* thioether bonds;
Ser^{d}-Lys^{d} are covalently linked *via* a lysinoalanine bond;
HO-Asp is β-hydroxyaspartic acid.

10. A method of preparation of a radiopharmaceutical composition, said composition comprising:
(i) the radiotracer as defined in any one of claims 1 or 7-9;
(ii) at least one radioprotectant as defined in claim 1 or 6;
(iii) a biocompatible carrier which comprises aqueous ethanol having an ethanol content of 0.1 to 10 % v/v;
in a form suitable for mammalian administration;
where said method of preparation comprises:
• carrying out the radiotracer purification method of steps (a)-(f) as defined in any one of claims 1 to 9;
(g) optionally diluting the purified [¹⁸F]-radiotracer from step (f) with a biocompatible carrier;
(h) aseptic filtration of the optionally diluted solution from step (g) to give said [¹⁸F] radiopharmaceutical composition.

11. The method of claim 10, wherein at least one of steps (b)-(f) or (b)-(h) is automated and where the automation is carried out using an automated synthesizer apparatus.

12. The method of claim 11, where said automated synthesizer apparatus comprises a single use cassette.

13. The method of claim 12, where the single use cassette comprises:
(i) a vessel containing the [¹⁸F]-radiotracer solution to be purified;
(ii) one or more reverse phase SPE cartridges;
(iii) a supply of the wash solution as defined in claim 1;
(iv) a supply of the elution solvent as defined in claim 1.

14. A single use cassette as defined in claim 12, for carrying out the automated method according to claim 11 or claim 12, which comprises:
(i) a vessel suitable for containing the [¹⁸F]-radiotracer solution to be purified;
(ii) one or more C8 or C18 reverse phase SPE cartridges;
(iii) a supply of the wash solution as defined in claim 1;
(iv) a supply of the elution solvent as defined in claim 1 wherein said elution solvent comprises 40-60 % v/v aqueous ethanol.

15. Use of the cassette as defined in Claim 14 fitted onto an automated synthesizer apparatus as defined in any one of claims 11 to 13, to carry out the method of purification of any one of claims 1 to 9, or the method of preparation of any one of claims 10 to 13.

## Patentansprüche

1. Verfahren zur Reinigung eines Radiotracers, umfassend die folgenden Schritte:
(a) Bereitstellung eines Radiotracers, der einen ¹⁸F-markierten aminoxyfunktionalisierten biologischen Targeting-Rest umfasst;
(b) Zugeben eines Strahlenschutzmittels zum Radiotracer, um eine Radiotracer-Lösung zu erhalten, die den Radiotracer in einem oder mehreren wässrigen wassermischbaren organischen Lösungsmitteln mit einem Gehalt an organischem Lösungsmittel von 5 bis 25 Vol.-% umfasst;
(c) Leiten der Radiotracer-Lösung von Schritt (b) durch eine Umkehrphasen-SPE-Kartusche, wobei der Radiotracer auf der SPE-Kartusche zurückgehalten wird;
(d) Waschen der SPE-Kartusche von Schritt (c) ein- oder mehrmals mit einer Waschlösung, die eine wässrige Lösung eines Strahlenschutzmittels in (einem) wassermischbaren organischen Lösungsmittel(n) mit einem Gehalt an organischem Lösungsmittel von 15 bis 25 Vol.-% umfasst;
(e) Waschen der SPE-Kartusche von Schritt (d) ein- oder mehrmals mit Wasser oder einer wässrigen Pufferlösung;
(f) Eluieren der gewaschenen SPE-Kartusche von Schritt (d) oder (e) mit einem Elutionslösungsmittel, das ein Strahlenschutzmittel in einer wässrigen Ethanollösung mit einem Ethanolgehalt von 35 bis 80 Vol.-% umfasst, wobei das Eluent gereinigten Radiotracer im Elutionslösungsmittel umfasst;
wobei jedes Strahlenschutzmittel unabhängig eines oder mehrere umfasst von: Ascorbinsäure; *para*-Aminobenzoesäure und Gentisinsäure und Salze davon mit einem bioverträglichen Kation.

2. Verfahren nach Anspruch 1, wobei das wassermischbare organische Lösungsmittel der Radiotracer-Lösung Acetonitril umfasst.

3. Verfahren nach Anspruch 1 oder Anspruch 2, wobei die Radiotracer-Lösung von Schritt (b) 0,5 bis 5 Vol.-% Ethanol umfasst.

4. Verfahren nach einem der Ansprüche 1 bis 3, wobei die SPE-Kartusche eine C18-SPE-Kartusche ist.

5. Verfahren nach einem der Ansprüche 1 bis 4, wobei das Elutionslösungsmittel von Schritt (f) 35 bis 70 Vol.-% wässriges Ethanol umfasst.

6. Verfahren nach einem der Ansprüche 1 bis 5, wobei das Strahlenschutzmittel 4-Aminobenzoesäure oder ein Salz davon mit einem bioverträglichen Kation umfasst.

7. Verfahren nach einem der Ansprüche 1 bis 6, wobei der BTM eine einzelne Aminosäure, ein 3-100-mer-Peptid, ein Enzymsubstrat, einen Enzymantagonisten, einen Enzymagonisten, einen Enzyminhibitor oder eine Rezeptor-bindende Verbindung umfasst.

8. Verfahren nach Anspruch 7, wobei der BTM einen Affibody™ umfasst.

9. Verfahren nach Anspruch 7, wobei der BTM ein 3-100-mer-Peptid umfasst, das aus Peptid A, Peptid B, Peptid C und Peptid D gemäß der nachfolgenden Definition ausgewählt ist:
(i) Peptid A = ein Arg-Gly-Asp-Peptid;
(ii) Peptid B = ein Arg-Gly-Asp-Peptid, welches das folgende Fragment umfasst 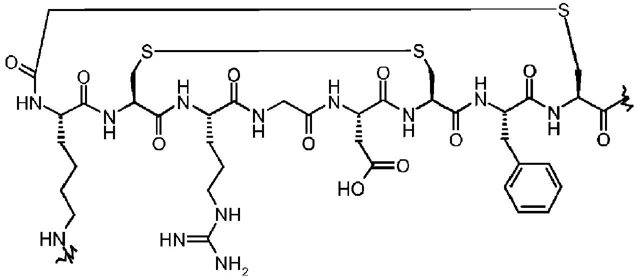
(iii) Peptid C = ein c-Met-bindendes cyclisches Peptid, das die folgende Aminosäuresequenz umfasst:
-Cys^{a}-X¹-Cys^{c}-X²-Gly-Pro-Pro-X³-Phe-Glu-Cys^{d}-Trp-Cys^{b}-Tyr-X⁴-X⁵-X⁶-,
wobei X¹ Asn, His oder Tyr ist;
X² Gly, Ser, Thr oder Asn ist;
X³ Thr oder Arg ist;
X⁴ Ala, Asp, Glu, Gly oder Ser ist;
X⁵ Ser oder Thr ist;
X⁶ Asp oder Glu ist;
und Cys^{a-d} jeweils Cysteinreste sind, sodass die Reste a und b sowie c und d cyclisiert sind, wodurch zwei getrennte Disulfidbindungen gebildet werden;
(iv) Peptid D = ein lanbiotisches Peptid der Formel:
wobei Xaa Arg oder Lys ist;
Cys^{a}-Thr^{a}, Ser^{b}-Cys^{b} und Cys^{c}-Thr^{c} *über* Thioetherbindungen kovalent gebunden sind;
Ser^{d}-Lys^{d} *über* eine Lysinoalanin-Bindung kovalent gebunden sind;
HO-Asp β-Hydroxyaspartinsäure ist.

10. Verfahren zur Herstellung einer radiopharmazeutischen Zusammensetzung, wobei die Zusammensetzung Folgendes umfasst:
(i) den Radiotracer nach einem der Ansprüche 1 oder 7-9;
(ii) wenigstens ein Strahlenschutzmittel nach Anspruch 1 oder 6;
(iii) einen bioverträglichen Träger, der wässriges Ethanol mit einem Ethanolgehalt von 0,1 bis 10 Vol.-% umfasst;
in einer für die Verabreichung an Säuger geeigneten Form;
wobei das Herstellungsverfahren Folgendes umfasst:
• Durchführen des Radiotracer-Reinigungsverfahrens der Schritte (a)-(f) nach einem der Ansprüche 1 bis 9;
(g) gegebenenfalls das Verdünnen des gereinigten [¹⁸F]-Radiotracers aus Schritt (f) mit einem bioverträglichen Träger;
(h) aseptische Filtration der gegebenenfalls verdünnten Lösung von Schritt (g), um die radiopharmazeutischen [¹⁸F]-Zusammensetzung zu erhalten.

11. Verfahren nach Anspruch 10, wobei wenigstens einer der Schritte (b)-(f) oder (b)-(h) automatisiert ist und wobei die Automatisierung unter Verwendung einer automatisierten Synthesizereinrichtung ausgeführt wird.

12. Verfahren nach Anspruch 11, wobei die automatisierte Synthesizereinrichtung eine Einwegkassette umfasst.

13. Verfahren nach Anspruch 12, wobei die Einwegkassette Folgendes umfasst:
(i) einen Behälter, der die zu reinigende [¹⁸F]-Radiotracer-Lösung enthält;
(ii) eine oder mehrere Umkehrphasen-SPE-Kartuschen;
(iii) einen Vorrat der Waschlösung nach Anspruch 1;
(iv) einen Vorrat des Elutionslösungsmittels nach Anspruch 1.

14. Einwegkassette nach Anspruch 12 zur Ausführung des automatisierten Verfahrens nach Anspruch 11 oder Anspruch 12, umfassend:
(i) einen Behälter, der dazu geeignet ist, die zu reinigende [¹⁸F]-Radiotracer-Lösung zu enthalten;
(ii) eine oder mehrere C8- oder C18-Umkehrphasen-SPE-Kartuschen;
(iii) einen Vorrat der Waschlösung nach Anspruch 1;
(iv) einen Vorrat des Elutionslösungsmittels nach Anspruch 1, wobei das Elutionslösungsmittel 40-60 Vol.-% wässriges Ethanol umfasst.

15. Verwendung der Kassette nach Anspruch 14, die auf einer automatisierten Synthesizereinrichtung nach einem der Ansprüche 11 bis 13 angebracht ist, zur Ausführung des Reinigungsverfahrens nach einem der Ansprüche 1 bis 9 oder des Herstellungsverfahrens nach einem der Ansprüche 10 bis 13.

## Revendications

1. Procédé de purification d'un radiotraceur qui comprend les étapes suivantes :
(a) la fourniture d'un radiotraceur qui comprend un fragment de ciblage biologique fonctionnalisé avec un aminoxy à marquage ¹⁸F ;
(b) l'ajout d'un radioprotecteur audit radiotraceur pour donner une solution de radiotraceur qui comprend ledit radiotraceur dans un ou plusieurs solvants organiques aqueux miscibles à l'eau à une teneur en solvant organique de 5 à 25 % en volume/volume ;
(c) le passage de la solution de radiotraceur provenant de l'étape (b) à travers une cartouche SPE à phase inversée, dans lequel le radiotraceur est retenu sur ladite cartouche SPE ;
(d) le lavage de la cartouche SPE provenant de l'étape (c) une ou plusieurs fois avec une solution de lavage qui comprend une solution d'un radioprotecteur dans un ou plusieurs solvants organiques aqueux miscibles à l'eau à une teneur en solvant organique de 15 à 25 % en volume/volume ;
(e) le lavage de la cartouche SPE provenant de l'étape (d) une ou plusieurs fois avec de l'eau ou une solution tampon aqueuse ;
(f) l'élution de la cartouche SPE lavée de l'étape (d) ou (e) avec un solvant d'élution qui comprend un radioprotecteur dans une solution aqueuse d'éthanol présentant une teneur en éthanol de 35 à 80 % en volume/volume, dans lequel l'éluant comprend un radiotraceur purifié dans ladite solution d'élution ;
dans lequel chaque radioprotecteur comprend indépendamment un ou plusieurs parmi : l'acide ascorbique ; l'acide *para*-aminobenzoïque ; et l'acide gentisique, et les sels de ceux-ci avec un cation biocompatible.

2. Procédé selon la revendication 1, dans lequel le solvant organique miscible à l'eau de la solution de radiotraceur comprend de l'acétonitrile.

3. Procédé selon la revendication 1 ou la revendication 2, dans lequel la solution de radiotraceur de l'étape (b) comprend 0,5 à 5 % en volume/volume d'éthanol.

4. Procédé selon l'une quelconque des revendications 1 à 3, dans lequel la cartouche SPE est une cartouche SPE en C18.

5. Procédé selon l'une quelconque des revendications 1 à 4, dans lequel le solvant d'élution de l'étape (f) comprend 35 à 70 % en volume/volume d'éthanol aqueux.

6. Procédé selon l'une quelconque des revendications 1 à 5, dans lequel le radioprotecteur comprend de l'acide 4-aminobenzoïque, ou un sel de celui-ci avec un cation biocompatible.

7. Procédé selon l'une quelconque des revendications 1 à 6, dans lequel le BTM comprend un seul acide aminé, un peptide de 3 à 100 mères, un substrat d'enzyme, un antagoniste d'enzyme, un agoniste d'enzyme, un inhibiteur d'enzyme ou un composé se liant à un récepteur.

8. Procédé selon la revendication 7, dans lequel le BTM comprend un Affibody™.

9. Procédé selon la revendication 7, dans lequel le BTM comprend un peptide de 3 à 100 mères qui est choisi parmi le Peptide A, le Peptide B, le Peptide C et le Peptide D tels que définis ci-dessous :
(i) Peptide A = un peptide Arg-Gly-Asp ;
(ii) Peptide B = un peptide Arg-Gly-Asp qui comprend le fragment 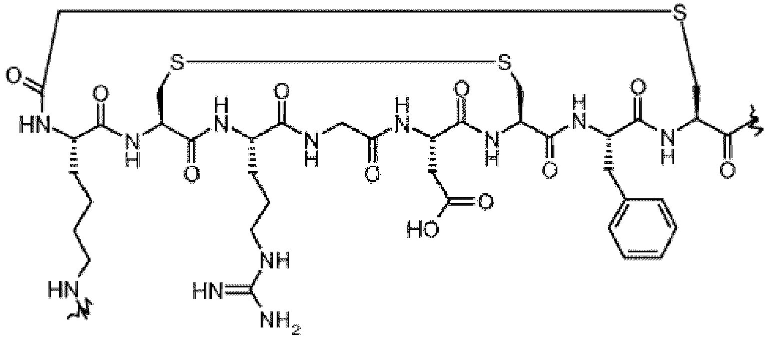
(iii) Peptide C = un peptide cyclique se liant à c-Met qui comprend la séquence d'acides aminés :
-Cys^{a}-X¹-Cys^{c}-X²-Gly-Pro-Pro-X³-Phe-Glu-Cys^{d}-Trp-Cys^{b}-Tyr-X⁴-X⁵-X⁶-
dans laquelle X¹ est Asn, His ou Tyr ;
X² est Gly, Ser, Thr ou Asn ;
X³ est Thr ou Arg ;
X⁴ est Ala, Asp, Glu, Gly ou Ser ;
X⁵ est Ser ou Thr;
X⁶ est Asp ou Glu ;
et Cys^{a-d} sont chacun des résidus cystéine tels que les résidus a et b ainsi que c et d sont cyclisés pour former deux liaisons disulfure séparées ;
(iv) Peptide D = un peptide lantibiotique de formule :
dans laquelle Xaa est Arg ou Lys ;
Cys^{a}-Thr^{a}, Ser^{b}-Cys^{b} et Cys^{c}-Thr^{c} sont liés par liaison covalente *via* des liaisons thioéther ;
Ser^{d}-Lys^{d} sont liés par liaison covalente *via* une liaison lysinoalanine ;
HO-Asp est l'acide β-hydroxyaspartique.

10. Procédé de préparation d'une composition radiopharmaceutique, ladite composition comprenant :
(i) le radiotraceur tel que défini dans l'une quelconque des revendications 1 ou 7 à 9 ;
(ii) au moins un radioprotecteur tel que défini dans la revendication 1 ou 6 ;
(iii) un support biocompatible qui comprend de l'éthanol aqueux présentant une teneur en éthanol de 0,1 à 10 % en volume/volume ;
sous une forme appropriée pour une administration aux mammifères ;
dans lequel ledit procédé de préparation comprend :
• la mise en oeuvre du procédé de purification de radiotraceur des étapes (a) à (f) tel que défini dans l'une quelconque des revendications 1 à 9 ;
(g) facultativement la dilution du [¹⁸F]-radiotraceur purifié provenant de l'étape (f) avec un support biocompatible ;
(h) la filtration aseptique de la solution facultativement diluée provenant de l'étape (g) pour donner ladite composition radiopharmaceutique à [¹⁸F].

11. Procédé selon la revendication 10, dans lequel au moins l'une des étapes (b) à (f) ou (b) à (h) est automatisée et dans lequel l'automatisation est effectuée en utilisant un appareil de synthèse automatique.

12. Procédé selon la revendication 11, dans lequel ledit appareil de synthèse automatique comprend une cassette à usage unique.

13. Procédé selon la revendication 12, dans lequel la cassette à usage unique comprend :
(i) un récipient contenant la solution de [¹⁸F]-radiotraceur à purifier ;
(ii) une ou plusieurs cartouches SPE à phase inversée ;
(iii) un approvisionnement en solution de lavage telle que définie dans la revendication 1 ;
(iv) un approvisionnement en solvant d'élution tel que défini dans la revendication 1.

14. Cassette à usage unique telle que définie dans la revendication 12, pour mettre en oeuvre le procédé automatisé selon la revendication 11 ou la revendication 12, qui comprend :
(i) un récipient approprié pour contenir la solution de [¹⁸F]-radiotraceur à purifier ;
(ii) une ou plusieurs cartouches SPE à phase inversée en C8 ou C18 ;
(iii) un approvisionnement en solution de lavage telle que définie dans la revendication 1 ;
(iv) un approvisionnement en solvant d'élution tel que défini dans la revendication 1 dans laquelle ledit solvant d'élution comprend 40 à 60% en volume/volume d'éthanol aqueux.

15. Utilisation de la cassette telle que définie dans la revendication 14 installée sur un appareil de synthèse automatique tel que défini dans l'une quelconque des revendications 11 à 13, pour mettre en oeuvre le procédé de purification selon l'une quelconque des revendications 1 à 9, ou le procédé de préparation selon l'une quelconque des revendications 10 à 13.
